(19) Europäisches Patentamt
European Patent Office
Office européen des brevets

(11) Publication number: 0 090 861 B1

(12) EUROPEAN PATENT SPECIFICATION

(45) Date of publication of patent specification: 09.09.87

(51) Int. Cl.$^4$: C 07 F 9/28, C 07 F 9/66, A 61 K 43/00, A 61 K 49/00, G 01 T 1/00

(21) Application number: 83900093.2

(22) Date of filing: 15.10.82

(86) International application number: PCT/US82/01482

(87) International publication number: WO 83/01450 28.04.83 Gazette 83/10

# (54) CATIONIC COMPOUNDS USEFUL FOR MAKING RADIODIAGNOSTIC AGENTS.

(30) Priority: 15.10.81 US 311770
02.08.82 US 404372

(43) Date of publication of application:
12.10.83 Bulletin 83/41

(45) Publication of the grant of the patent:
09.09.87 Bulletin 87/37

(84) Designated Contracting States:
AT BE CH DE FR GB LI LU NL SE

(56) References cited:
EP-A-0 038 756
US-A-4 133 872
US-A-4 247 534
US-A-4 363 793

Journal of Nuclear Medicine, Volume issued October, 1981, Deutsch et al, see pages 897-907.

(73) Proprietor: E.I. DU PONT DE NEMOURS AND COMPANY
1007 Market Street
Wilmington Delaware 19898 (US)

(72) Inventor: SUBRAMANYAM, Vinayakam
40 Crystall Holl
Westwood, MA 02090 (US)
Inventor: LINDER, Karen E.
84 Hudson Street
Somerville, MA 02143 (US)

(74) Representative: Sandmair, Kurt, Dr. et al
Patentanwälte Schwabe, Sandmair, Marx
Stuntzstrasse 16
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 090 861 B1

**Description**

Field of the Invention

The present invention relates to cationic radiodiagnostic agents and, in particular, to water soluble cationic ligands useful as intermediates for producing 99mTc-labelled cationic radiodiagnostic agents, and kits for preparing such 99mTc-labelled cationic radiodiagnostic agents.

Background of the Invention

Various complexes of monodentate and bidentate ligands with technetium have been made and studied. These complexes generally were made for use in studies to determine the various oxidation states of technetium and for other research regarding the structure of such complexes and metal-coordination chemsitry. Such studies have been reported in, for instance, *Chemistry and Industry,* pp. 347—8 (March 26, 1960); *J. Inorg. Nucl. Chem.,* Vol. 28, pp. 2293—96 (1966); *Aust. J. Chem.,* 23, pp. 453—61 (1970); *Inorganic Chem.,* Vol. 16, No. 5, pp. 1041—48 (1977); *J. Inorg. Nucl. Chem.,* Vol. 39, pp. 1090—92 (1977); and *J. C. S. Dalton,* pp. 125—30 (1976).

Recently, in a presentation to the American Pharmaceutical Association, and also in EP—A—0 038 756, E. A. Deutsch disclosed that certain complexes of DIARS, i.e.

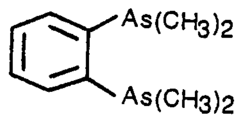

I

and Tc-99m, and certain complexes of DMPE, i.e. $(CH_3)_2PCH_2CH_2P(CH_3)_2$ and Tc-99m, may be useful as radiodiagnostic agents for myocardial or hepatobiliary imaging. $[99mTc\text{-}(DMPE)_2Cl_2]^+$ and $[99mTc\text{-}(DIARS)_2Br_2]^+$ were prepared by Deutsch by heating in an open flask a reaction mixture containing the appropriate hydrogen halide in aqueous alcohol solution, 99mTc-sodium pertechnetate, and ortho-phenylenebis(dimethylarsine), i.e. DIARS, or bis-(1,2-dimethylphosphino)ethane, i.e. DMPE. The reaction was reported to take about 30 minutes. The labelled complex was then purified by chromatographic methods involving ion exchange columns.

The labelled complex produced according to the procedure of Deutsch has several practical disadvantages. The procedure requires handling several ingredients including an organic solvent to make the reaction mixture and then purifying the resulting radiolabelled complex by chromatography. Each of these handling steps can contaminate the system and final product. The purification step further requires additional time for preparation of the final product. These steps require a skilled technician and are performed at the site of use, just prior to use. Thus, a complex, time consuming chemical preparation is required during which sterility of ingredients and containers is difficult to maintain. Thus, to assure freedom from contamination, a final sterilization step is required, which further adds to preparation time. Because Tc-99m has a short half-life, lengthy preparation methods are undesirable. Thus, the complexity of the preparation, both with regard to maintaining sterile conditions and to purification of the 99mTc-labelled complex make the Deutsch procedure undesirable.

It would be highly desirable to have a sterilized kit with all the necessary materials prepared by the manufacturer, to which only the Tc-99m need be added at the site of use to produce the desired labelled complex directly in high enough yield to obviate the need for purification. It would also be desirable for the kit materials to be in a closed container or vial, pre-sterilized, so that the only step to be performed at the site of use would be the addition of the radionuclide. To increase stability and shelf-life of the kit, it would be highly desirable that the materials be readily lyophilized, preferably from an aqueous solution.

By achieving the desirable features outlined above, a convenient-to-use heart imaging radiopharmaceutical agent would be provided that is capable of concentrating in healthy heart tissue to provide a negative image of an infarct, damaged or ischemic tissue.

Summary of the Invention

The present invention provides an acid salt of a mono or polydentate ligand that is water soluble, stable in a lyophilized state, and is capable of binding with Tc-99m to form a cationic complex. The acid salt is represented by the formula:

(A) 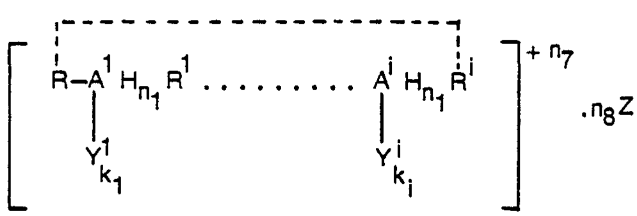

wherein:

i is an integer from 1 to 6;

R, $R^1$, $R^2$, $R^3$, $R^4$, $R^5$ and $R^6$ are each independently selected from hydrogen, alkyl, aryl, alkylaryl, arylalkyl, monocycloalkyl and polycycloalkyl, or R plus $R^i$ may be taken together to form a cyclic compound;

$y^1$, $y^2$, $y^3$, $y^4$, $y^5$ and $y^6$ are independently selected from alkyl, aryl, alkylaryl, arylalkyl, monocycloalkyl and polycycloalkyl;

$A^1$, $A^2$, $A^3$, $A^4$, $A^5$ and $A^6$ are the same or different donor atoms, each having a free-electron pair available for accepting a proton to provide a charged ligand or for complexing with Tc-99m or Tc-99c to form a cationic complex;

Z is an anion;

$k_1$, $k_2$, $k_3$, $k_4$, $k_5$ and $k_6$ are each independently zero or one;

$n_1$, $n_2$, $n_3$, $n_4$, $n_5$ and $n_6$ are independently 0 or 1; and

$n_7$ and $n_8$ are integers from 1 to 6 where

$$n_7 = \sum_{i=1}^{6} n_i .$$

and the charge represented by $n_8Z$ is equal in magnitude and opposite in sign to $+n_7$; or

(B) 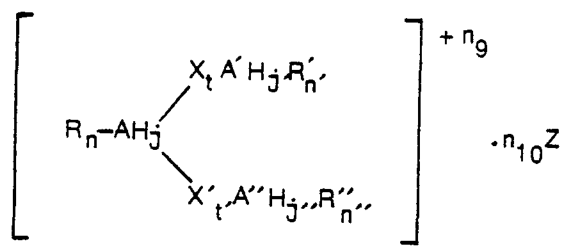

wherein:

R, R′ and R″ are independently selected from hydrogen, alkyl, aryl, alkylaryl, arylalkyl, monocycloalkyl and polycycloalkyl;

A, A′ and A″ are independently selected from the group of donor atoms having a pair of electrons available for accepting a proton to provide a charged ligand or for complexing with Tc-99m or Tc-99 to form a cationic complex;

X and X′ are alkyl groups;

t and t′ are independently 0 or 1;

j, j′ and j″ are independently 0 or 1; n, n′ and n″ are independently the integer 1 or 2;

Z is the same as defined above;

$n_9$ and $n_{10}$ are integers selected from 1 to 3;

where $n_9 = j + j' + j''$ and the charge represented by $n_{10}Z$ is equal in magnitude and opposite in sign to $+n_9$.

The acid salts of the present invention having the above formulas are normally solid compounds, water-soluble, readily lyophilized, and capable of reducing pertechnetate and binding with technetium to form stable cationic complexes. For purposes of this invention a "stable cationic complex" means a cationic complex that has a sufficiently long life to administer it as a radiopharmaceutical and obtain the desired radioscintigraphic image.

The R's and X's in formulas (A) and (B) are alkyl radicals having 1 to 6 carbon atoms such as methyl or ethyl, and arylalkyl and aryl radicals such as benzyl or phenyl. When more than one R group is attached to the same donor atom, the R groups so attached can be the same or different. The novel ligand acid salts of the present invention are useful for preparing radiopharmaceutical compositions containing cationic complexes of Tc-99m. Such cationic technetium-labelled complexes are useful in radiodiagnostic tests in connection with myocardial or hepatobiliary tissues.

The useful cationic Tc-99m complexes are made by reacting the novel ligand acid salts of this invention with 99mTc-pertechnetate in the presence of a suitable complex formation assisting anion. In one embodiment, the reaction is carried out in the presence of halide ions, and of these the chloride is most preferred. In another embodiment, the reaction is carried out in the presence of pseudo-halogen anions, and of these the reaction product with thiocyanate ion is particularly useful. In yet another embodiment, the reaction is carried out in the presence of hydroxide anions.

Detailed Description of the Invention

The water-soluble ligand acid salts of the present invention can be prepared from a wide variety of monodentate and polydentate ligands. Typical examples of such ligands include, for instance, aryl

compounds having arsenic, phosphorus, nitrogen, sulfur, oxygen, selenium, tellurium, or any combination of them, substituted ortho to each other. For example, o-phenylene compounds having the structure:

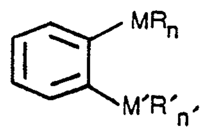

II

in which M and M' are arsenic, phosphorus, nitrogen, sulfur, oxygen, selenium, tellurium, or any combination of them, n and n' are independently 1 or 2 depending upon the particular donor atom used for M and M', and R and R' are independently hydrogen, or an organic group, preferably an alkyl group having 1 to 6 carbon atoms, an aryl group such as phenyl. Additional examples of suitable ligands include bidentate cis-tetraethylene ligands of the formula:

$$R'_{n'}M'—CX'_2CX_2—MR_n \quad \text{III}$$

in which M, M', R, and R' are as defined above, n and n' are independently 1 or 2 depending upon the particular donor atom used for M and M', and X and X' are independently selected from hydrogen, halide, or alkyl groups having 1 to 6 carbon atoms. Further examples of suitable ligands include those having the formula:

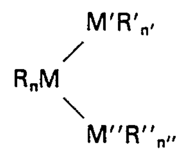

wherein M, M', R, and R', are as defined above, M'' is independently selected from arsenic, phosphorus, nitrogen, sulfur, oxygen, selenium, and tellurium, n is 0 or 1, n' and n'' are independently 0, 1 or 2, and R'' is independently selected from hydrogen, halide or an organic radical, preferably an alkyl radical having 1 to 6 carbon atoms, an aryl radical such as phenyl.

Particularly preferred ligands for the practice of this invention are the bis-dialkylphosphinoethanes and their substituted derivatives, including, for example,

1,2-bis(dimethylphosphino)ethane,
1,2-bis(di(trifluoromethyl)phosphino)ethane,
1,2-bis(dimethylphosphino)-1,1-difluoroethane,
1,2-bis(dimethylphosphino)-1-fluoroethane,
1,2-bis(dimethylphosphino)propane,
1,2-bis(di(trifluoromethyl)phosphino)-1,1,2,2-tetrafluoroethane,
1,2-bis(di(trifluoromethyl)phosphino)propane,
2,3-bis(di(trifluoromethyl)phosphino)butane,
1,2-bis(di(trifluoromethyl)phosphino)butane,
1,3-bis(dimethylphosphino)butane,
1,3-bis(dimethylphosphino)propane,
1,3-bis(di(trifluoromethyl)phosphino)propane,
1,2-bis(dimethylphosphino)-1,1-dichloro-2,2-difluoroethane,

and similar compounds wherein the phosphorus is replaced by nitrogen, arsenic, sulfur, oxygen, selenium, tellurium, or another atom having a free electron pair.

Other useful ligands include the alkylaminobis(difluorophosphine), i.e., $RN(PF_2)_2$, ligands where R is an organic group, preferably an alkyl group having 1 to 6 carbon atoms, an aryl group such as phenyl; and the o-phenylene compound such as, for example,

ortho-phenylenebis(diarsine),
ortho-phenylenebis(dimethylarsine),
ortho-phenylenediamine,
ortho-phenylenebis(dimethylamine),
ortho-phenylenebis(diphosphine), and
ortho-phenylenebis(dimethylphosphine).

Additional ligands suitable for use in the present invention are those described by Nozzo et al., in *J. Amer. Chem. Soc., 101,* p. 3683 (1979) and by Wilson et al., *J. Amer. Chem. Soc., 100,* p. 2269 (1978).

Any donor element can be used in the ligand in accord with this invention provided that it is an atom having a free-electron pair avilable for accepting a proton to provide a charged ligand and further provided that it has the capability of complexing with technetium (Tc-99 or Tc-99m) to form a cationic complex in the presence of suitable anions. Suitable such elements include, for instance, phosphorous (P), arsenic (As), nitrogen (N), oxygen (O), sulfur (S), antimony (Sb), selenium (Se) and tellurium (Te). Preferred elements are P and As.

The acid salt of the ligand is prepared by mixing the ligand with an acid preferably having a parenterally acceptable hydrophilic anion and able to protonate the ligand in a suitable solvent. After evaporation of the solvent, the ligand acid salt is purified by recrystallization from the same solvent or another convenient solvent.

Any acid having a hydrophilic anion and capable of protonating the ligand can be used to produce the ligand acid salt. Preferably, such acids have an oxygen containing hydrophilic anion. Examples of such acids suitable for the practice of this invention include, for instance, sulfuric acid, nitric acid, phosphoric acid, tetrafluoroboric acid, oxalic acid, trichloroacetic acid, perchloric acid, hexafluorophosphonic acid, and boric acid. For use in humans, toxicologically acceptable acids, i.e. acids comprising toxicologically acceptable anions, are preferred.

The cationic technetium complexes useful in methods for radiodiagnostic treatments are prepared by mixing in an aqueous or alcoholic solution and the ligand acid salt and 99mTc-pertechnetate in the presence of a suitable complex formation assisting anion and heating the mixture to form the cationic complex. Suitable complex formation assisting anions include, for instance, halide ions; pseudo-halogen ions such as the thiocyanato- ion, cyano- ion, cyanato- ion, selenocyanato- ion, fulminato- ion, and the isoforms of these ions, and the azido- ions and hydroxide ions; which aid technetium in achieving a stable complex with the particular ligand. Preferably, the ligand acid salt is lyophilized and is contained in a sealed, sterilized vial prior to adding the pertechnetate. The pertechnetate solution can then be injected into the vial under aseptic conditions to maintain sterility. The vial is generally heated and maintained at an elevated temperature for sufficient time to form a complex of the ligand with technetium. The vial should be heated to at least 80°C for 30 minutes or more. Preferably, the vial is heated to 100°C or more, and more preferably to a temperature in the range of 130°C to 150°C. At about 150°C, the reaction can be completed in about five to ten minutes, depending upon the choice and concentrations of the reactants.

It has been found that kits for the preparation of the cationic technetium complex are improved by the addition of a polyhydroxy-compound to the reaction mixture. The use of the polyhydroxy-compound, for reasons not fully understood, results in a more consistent yield of the cationic technetium complex. Preferred polyhydroxy-compounds include, for example, Hetastarch (hydroxyethyl starch), mannitol, glycerol, D-mannose and sorbitol.

The ligand acid salt of the present invention is preferably supplied in a radiopharmaceutical preparation kit comprising a sterilized unit (or multiple) dose vial containing the purified ligand acid salt. About 50 mCi of 99mTc-pertechetate in saline is injected aseptically into the vial and the mixture heated to form the labelled cationic complex. After cooling, the resulting radiopharmaceutical preparation may be adjusted for pH and is ready for use. Typically, when the pH is adjusted, it is adjusted into the range of 4.0 to 9.0, and preferably to physiological pH.

The kit preferably comprises a vial containing a lyophilized mixture obtained from an aqueous solution of the ligand acid salt, a polyhydroxy-compound, and an acid or buffer to control the pH. Preferably the pH is in the range of 1.0 to 3.0, and more preferably in the range of 1.5 to 2.5, when using halide anions. The most desirable pH will, of course, vary depending upon the particular ligand and salt thereof being used, the anion, and upon their concentrations. For instance, when it is desired to form the cationic technetium complex in the presence of hydroxide ions as the anion, a pH greater than 9.0 is desirable. A halide or other salt can also be added to the solution to be lyophilized.

To image the heart of a mammal, in-vivo, a radiopharmaceutical preparation in accord with the invention, having a suitable quantity of radioactivity for the particular mammal, is injected intravenously into the mammal. The mammal is positioned under a scintillation camera in such a way that the heart is covered by the field of view. High quality images of the heart are obtained analogous to those seen in clinical studies using Thallium-201.

In order to obtain high quality images the yield of radioactive labelled cationic technetium complex should preferably be greater than 70% after reconstituting the lyophilized mixture and labelling. Lower yields will result in poorer image quality and undesirable purification steps will be required to produce high quality images.

This invention will be further illustrated by the examples that follow:

Example 1

Preparation of 1,2-Bis(dimethylphosphino)ethane-bis(tetrafluoroborate), i.e. $(DMPEH)_2{}^{2+} \cdot 2BF_4{}^-$

Place 210 mg of 1,2-bis(dimethylphosphino)ethane in a 50 ml round-bottomed flask maintained under a nitrogen atmosphere, and dissolve it in 10 ml of ethanol. Add 0.5 ml of a 49% solution of tetrafluoroboric acid. After 15 minutes, remove the solvent in a rotary evaporator and recrystallize the product from 15 ml of ethanol. Filter and dry under vacuum. 406 mg of a crystalline solid is obtained, which melts at 199.5—210°C.

Example 2

Preparation of 1,2-Bis(dimethylphosphino)ethane bis-bisulfate, i.e. $DMPEH_2{}^{2+} \cdot 2HSO_4{}^-$ or $DMPE \cdot 2H_2SO_4$

Dissolve 470 mg of DMPE in 10 ml of ethanol in a 50 ml round-bottomed flask maintained under a nitrogen atmosphere. From a glass syringe, add, with stirring, 0.34 ml of concentrated sulfuric acid. After 10 minutes, filter the precipitate and recrystallize it from 10 ml of methanol. Filter and dry under vacuum. 920 mg of a crystalline solid is obtained, which melts at 135—136.5°C. Structure and purity of the compound

was confirmed by its infra-red and nuclear magnetic resonance spectra and elemental analysis.

Example 3

Propylene glycol — DMPE·$2H_2SO_4$ Kit

Prepare a solution of 80.5 mg of DMPE-bis(bisulfate) and 525 mg sodium chloride in 26.25 ml distilled water. Add 43.75 ml of propane-1,2-diol to it with stirring, followed by 0.4 ml of 2 N hydrochloric acid to adjust the pH from 2.46 to 1.92. Dispense 2 ml of this bulk preparation into each of 30 10-cc vials. Purge each vial with a steady stream of nitrogen gas for 20 seconds and crimp seal using a Teflon®-coated stopper. Inject 0.5 ml physiological saline containing approximately 10 mCi 99mTc-pertechnetate into the vial and place it in a steam autoclave preheated to 100°C. Set the temperature control to 135°C and when that temperature is reached, maintain it for 15 minutes. Allow the system to cool to 100°C and remove the vial from the autoclave. Evaluation by thin layer chromatography (TLC) shows that greater than 95% of the radioactivity is in the form of $[99mTcCl_2(DMPE)_2]^+$, the structure and charge of the complex having been confirmed by elemental analysis, infra-red and nuclear magnetic resonance spectra of the Tc-99 analogue, by electrophoretic mobility and high performance liquid chromatographic analysis (HPLC).

Example 4

Ethanol — DMPE Kit (Prior Art)

Prepare a saturated solution of sodium chloride in ethanol by dissolving 117 mg of sodium chloride in 20 ml of degassed ethanol. Add 11.1 μl of liquid DMPE to this solution and adjust its pH to approximately 2.25 by adding concentrated hydrochloric acid. Dispense 5 ml into a 10 cc vial and crimp seal it using a Teflon®-coated stopper. Inject approximately 5 mCi of 99mTc-pertechnetate eluate into the vial and place it in a boiling water bath for 1 hour. HPLC analysis shows a yield of greater than 80% of the $[99mTcCl_2(DMPE)_2]^+$ complex.

Example 5

Mannitol — DMPE·$2H_2SO_4$ Kit

Dissolve 1 g mannitol, 150 mg sodium chloride, and 46 mg DMPE-bis(bisulfate) in 10 ml deoxygenated physiological saline solution [0.15 Molar]. Adjust the pH of the solution to 1.4 by adding the required volume of 2 N hydrochloric acid. Dispense 1 ml of the solution into each of several 10 cc vials, flushing each with nitrogen gas for 20 seconds, closing with a Teflon®-coated stopper and crimp-sealing it.

Labelling Procedure A

Inject 50 mCi of 99mTc-pertechnetate in 0.5 ml physiological saline into each of several vials and place them in an oil bath, preheated and maintained at 150° ± 5°C, for 5—10 minutes. HPLC analyses, as in Example 4, show yields of 90 to 100%.

Labelling Procedure B

Inject 50 mCi of 99mTc-pertechnetate in 0.5 ml physiological saline into each of several vials and place them in a steam autoclave preheated to 100°C. Set the temperature control to 135°C, and when that temperature is achieved, maintain it for 20 minutes. Allow the system to cool to 100°C and remove the vials. HPLC analyses, as in Example 4, show yields of 95 to 100%.

Example 6

Mannitol — DMPE·$2H_2SO_4$ Kit

Dissolve 5 g mannitol and 230 mg DMPE-bis(bisulfate) in about 35 ml low-oxygen distilled water, and adjust the pH of the solution to 1.0 with 3 N sulfuric acid. Under cover of nitrogen, and with stirring, add low-oxygen distilled water gravimetrically, to a solution weight of 50 g. Dispense 1 ml of this solution into each of several 10 cc vials. Freeze-dry in keeping with procedures well-known to those skilled in the art, stoppering under nitrogen. Reconstitute each vial with 1 ml of physiological saline containing about 10—20 mCi 99mTc-pertechnetate. Utilizing techniques similar to those of Example 3, autoclave for 30 minutes at 135°C. TLC analyses, as in Example 3, show yields consistently greater than 95%.

Example 7

Glucoheptanoic acid — DMPE·$2H_2SO_4$ Kit

Dissolve 115 mg DMPE-bis(bisulfate) and 4.35 grams glucoheptanoic acid in 45 ml deoxygenated physiological saline. Prepare kits by dispensing 2 mls. of this bulk solution into each of several 10 cc vials; close under nitrogen with Teflon®-coated stoppers and crimp-seal them. Inject 0.5 ml of physiological saline containing approximately 5—10 mCi 99mTc-pertechnetate and heat for 6 minutes in an oil bath preheated and maintained at 150°C. Yield of $[99mTcCl_2(DMPE)_2]^+$ based on TLC analysis as in Example 3 is > 90%.

Example 8

DMPE·$2H_2SO_4$ — Sodium Chloride Kits

Dissolve 0.439 g of sodium chloride in 45 ml 2N sodium hydroxide solution and adjust the pH to 1.6

using concentrated hydrochloric acid. Add 115 mg bis (dimethylphosphino)ethane-bis-bisulfate, prepared as in Example 2, to the above solution, and dissolve it by stirring. Prepare several kits by dispensing 2 ml into each of several 10 cc vials, closing them under nitrogen with Teflon®-coated stoppers and crimp-sealing them. Inject 0.5 ml of normal physiological saline solution containing 5—10 mCi 99mTc-pertechnetate into each kit and heat in an oil bath, pre-heated and maintained at 150°C, for 6 minutes. The yield of $[99mTcCl_2(DMPE)_2]^+$ (based upon HPLC) is greater than 90%.

Example 9

Sucrose — $DMPE \cdot 2H_2SO_4$ Kit

Dissolve 4.6 mg of bis(dimethylphosphino)ethane-bis-bisulfate, prepared as in Example 2, and 430 mg sucrose in 2 ml physiological saline solution, adjust to pH 1.81 with hydrochloric aid. Close under nitrogen with a Teflon®-coated stopper, and crimp-seal. Inject 0.5 ml physiological saline containing 5—10 mCi 99mTc-pertechnetate eluate.

a) After autoclaving for 20 minutes at 135°C, the yield of $[99mTcCl_2(DMPE)_2]^+$ based upon TLC analysis as in Example 3, is 44%.

b) With extended autoclaving, the yield of $[99mTcCl_2(DMPE)_2]^+$ can be increased to at least 80%.

Example 10

Sorbitol — $DMPE \cdot 2H_2SO_4$ Kit

Dissolve 4.6 mg of bis(dimethylphosphino)ethane-bis-bisulfate, prepared as in Example 2, and 200 mg sorbitol in 2 ml physiological saline; adjust to pH 1.81 with hydrochloric acid. Close under nitrogen with a Teflon®-coated stopper, and crimp-seal. Add 0.5 ml physiological saline containing 5—10 mCi 99mTc-pertechnetate eluate. After autoclaving for 20 minutes at 135°C, the conversion to $[99mTcCl_2(DMPE)_2]^+$ is greater than 95%, by TLC analysis as in Example 3.

Example 11

Glycerol — $DMPE \cdot 2H_2SO_4$ Kit

Duplicate the procedure of Example 10 in every respect but the following: substitute glycerol for sorbitol. As in Example 10, the conversion to $[99mTcCl_2(DMPE)_2]^+$ is greater than 95%, by TLC analysis as in Example 3.

Example 12

Mannose — $DMPE \cdot 2H_2SO_4$ Kit

Duplicate the procedure of Example 10 in every respect but the following: substitute mannose for sorbitol. As in Example 10, the conversion to $[99mTcCl_2(DMPE)_2]^+$ is greater than 95%, by TLC analysis as in Example 3.

Example 13

Hydroxyethyl Starch — $DMPE \cdot 2H_2SO_4$ Kit

Duplicate the procedure of Example 10 in every respect but the following: substitute 600 μl Volex® (hydroxyethyl starch, 6% (W/V) in physiological saline, available from McGaw Laboratories) for sorbitol. As in Example 10, the conversion to $[99mTcCl_2(DMPE)_2]^+$ is greater than 95%, by TLC analysis as in Example 3.

Example 14

Mannitol — $DMPE \cdot 2H_2SO_4$ (Lyophilized Kit): $[99mTc\text{-}(DMPF)_3]^+$ Complex Formation

Dissolve 5 g mannitol and 115 mg DMPE-bis(bisulfate) in about 35 ml low-oxygen distilled water, and adjust the pH to 1.0 with 3N sulphuric acid. Under cover of nitrogen and with stirring add low-oxygen distilled water gravimetrically to a solution weight of 50 g. Dispense 1 ml of this solution into each of several 10 ml vials. Freeze-dry in keeping with procedures well-known to those skilled in the art, stoppering under nitrogen. Reconstitute each of the vials with 1 ml of physiological saline containing about 10—20 mCi of 99mTc-pertechnetate, and add 0.15 ml 1N sodium hydroxide solution. The pH is about 12. Utilizing techniques similar to those of Example 3, autoclave for 30 minutes at 135°C. TLC analyses show almost 95% yield of $[99mTc(DMPE_3]^+$, the structure and charge of the complex having been confirmed by elemental analysis, infra-red and nuclear magnetic resonance spectra of the Tc-99 analogue, and by electrophoretic mobility.

Example 15

Preparation of 99mTc-Thiocyanato — DMPE Complexes

a) To a 99mTc-labelled preparation of Example 6 is added 0.3 ml 0.5N sodium hydroxide containing 25 mg potassium thiocyanate. After heating for 30 minutes in a boiling water bath, HPLC analyses shows a labelled complex distinguishable from the products of Example 5 and Example 14, but which has similar cationic characteristics by electrophoresis.

b) To the freeze-dried (but not as yet labelled) preparation of Example 6 is added 1 ml of physiological saline containing 10—20 mCi 99mTc-pertechnetate and 0.3 ml 0.5N sodium hydroxide containing 25 mg potassium thiocyanate. After autoclaving for 30 minutes at 135°C as in Example 6, HPLC analysis reveals a

labelled complex distinguishable from the products of Examples 5, 14 or 15a, but which has similar cationic characteristics by electrophoresis.

Example 16

Imaging of Rabbit Heart Using Tl-201 (Prior Art)

2 mCi of Thallium-201 (as thallous chloride in physiological saline containing 0.9% benzyl alcohol) is injected intravenously into a 2.5 Kg male New Zealand Albino rabbit. The rabbit is positioned under a Searle Pho-Gamma scintillation camera in such a way that the heart and lung area are covered by the field of view. Approximately 10 minutes after injection, sufficient counts are accumulated to produce an image of the heart analogous to that seen in clinical studies of humans.

Example 17

Imaging of Rabbit Heart Using 99mTc-labelled Products with ≥ 80% Yield of Desired Labelled Complex

Greater than 1 mCi of the 99mTc-labelled product of Example 3 is injected into a rabbit and imaged as in Example 16. The quality and appearance of the heart image is similar to that obtained in Example 16.

The product of any one of Examples 4, 5, 6, 7, 8, 9b, 10, 11, 12, 13 or 14 produces similar results when injected into a rabbit as described above.

Example 18

Imaging of Baboon Heart Using 99mTc-labelled Products with ≥ 80% of Desired Labelled Complex

Greater than 10 mCi of any one of the 99mTc-labelled products of Example 17 is injected intravenously into an adult baboon positioned under a scintallation camera as was the rabbit in Example 17. Excellent quality images of the heart are obtained, which are equivalent to those characteristically obtained with Tl-201 in humans.

Example 19

Visualization of Hepatobiliary Transit with 99mTc-labelled Disofenin (Prior Art)

A lyophylized vial of HEPTATOLITETM (New England Nuclear Corporation's brand of Technetium Tc99m Disofenin) is labelled with 99mTc-pertechnetate in accordance with manufacturer's directions. At least 1 mCi of the labelled preparation is injected intravenously into a 2.5 Kg male New Zealand Albino rabbit. The rabbit is positioned under a Searle Pho-Gamma scintillation camera in such a way that the liver and gastro-intestinal tract are within the field of view. Sequential images taken from the time of injection demonstrate an initial liver uptake with gradual visualization of the gall bladder and gastro-intestinal tract, analogous to the diagnostically efficacious results obtained in clinical studies of normal healthy humans.

Example 20

Visualization of Hepatobiliary Transit with 99mTc-Thiocyanato- Complexes

Greater than 1 mCi of the 99mTc-Thiocyanato-DMPE complex from either Example 15a or 15b is injected into a rabbit as in Example 19. Sequential images of hepatobiliary transit reveal at least as rapid passage as that in Example 19, with comparable image quality of the liver, gall bladder and gastro-intestinal tract.

**Claims**

1. A normally solid, hydrophilic compound capable of binding with Tc-99m to form a cationic complex and having a formula selected from:

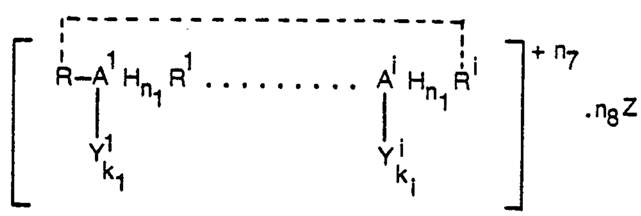

(A)

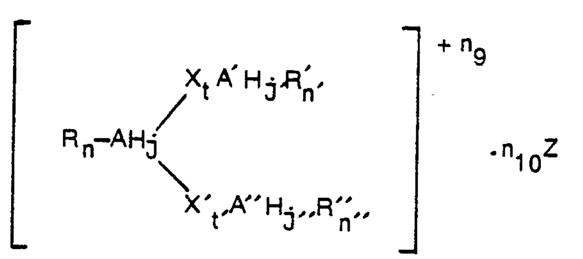

(B)

wherein:

R, R', R'', $R^1$, $R^2$, $R^3$, $R^4$, $R^5$ and $R^6$ are each selected from hydrogen, alkyl having 1—6 C-atoms, aryl, alkylaryl, arylalkyl, monocycloalkyl and polycycloalkyl; and R plus R' in formula (A) may be taken together to form a cyclic compound;

A, A', A'', $A^1$, $A^2$, $A^3$, $A^4$, $A^5$ and $A^6$ are donor atoms, each having a free electron pair available for acccepting a proton to provide a charged ligand and having the capability of complexing with Tc-99m to form a cationic complex and are independently selected from P, As, N, O, S, Sb, Se and Te;

$Y^1$, $Y^2$, $Y^3$, $Y^4$, $Y^5$ and $Y^6$ are each independently selected from hydrogen; or alkyl, aryl, alkylaryl, arylalkyl, monocycloalkyl and polycycloalkyl;

X and X' are alkyl groups having 1—6 C-atoms;

Z is an anion;

i is an integer from 1 to 6;

j, j' and j'' are each independently 0 or 1;

$k_1$, $k_2$, $k_3$, $k_4$, $k_5$ and $k_6$ are each independently 0 or 1;

t and t' are independently 0 or 1;

n, n' and n'' are each independently the integer 1 or 2;

$n_1$, $n_2$, $n_3$, $n_4$, $n_5$ and $n_6$ are independently 0 or 1;

$n_7$ and $n_8$ are each an integer from 1 to 6;

$n_9$ and $n_{10}$ are each an integer from 1 to 3.

2. The compound of claim 1 wherein Z is derived from sulfuric acid, phosphoric acid, perchloric acid, nitric acid or boric acid.

3. The compound of claim 1 having the formula:

$$R(AHjR'n')_n^{+n}\ .\ n''Z$$

wherein R is ethyl or phenyl, A is phosphorus or arsenic, R' is an alkyl group having from 1 to 6 carbon atoms, Z is an anion, j is 0 or 1; n, n' and n'' are 1 or 2.

4. The compound of claim 3 wherein Z is bisulfate, biphosphate, or tetrafluoroborate.

5. The compound of claim 1 having the formula:

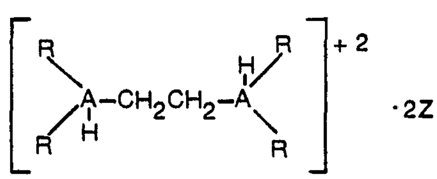

wherein

Z is an anion;

A is P or As; and

each R is independently H, an alkyl group having from 1 to 6 carbon atoms, or phenyl.

6. The compound of claim 5 wherein Z is bisulfate, biphosphate, nitrate, tetrafluoroborate or perchlorate.

7. The compound of claim 5 wherein A is phosphorus.

8. The compound of claim 5 wherein A is phosphorus and R is methyl or ethyl.

9. The compound of claim 5 having the formula:

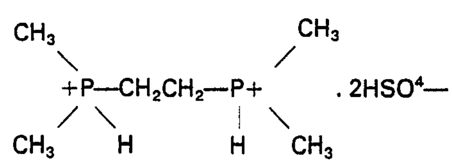

10. The compound of claim 1 having the formula:

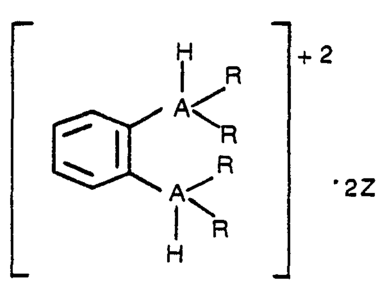

wherein A is P or As; and each R is independently H, or an alkyl group having from 1 to 6 carbon atoms and Z is an anion.

11. The compound of claim 10 wherein Z is bisulfate, biphosphate, nitrate, tetrafluoroborate or perchlorate.

12. The compound of claim 10 wherein A is phosphorus.

13. The compound of claim 10 wherein A is phosphorus and R is methyl or ethyl.

14. The compound of claim 10 having the formula:

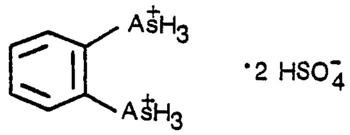

15. A kit for preparing a radiopharmaceutical preparation comprising a sealed, sterilized vial containing a compound according to any one of the preceding claims.

16. The kit of claim 15 wherein said vial further contains a halide ion or a thiocyanato, cyano, cyanato, selenocyanato, fulminato ion and the isoforms of these ions, an azido-ion or a hydroxide ion.

17. A method for making a cationic complex comprising Tc-99m for radioscintigraphic imaging, said method comprising admixing the compound of claim 1 with an aqueous solution containing Tc-99m in the presence of a halide ion or a thiocyanato, cyano, cyanato, selenocyanato, fulminato ion and the iso-form of these ions, or an azido or hydroxide ion, and heating the admixture to form a stable complex.

18. The method of claim 17 carried out under aseptic conditions and in a closed system.

19. The method of claim 18 wherein said compound is contained in a sterile, sealed vial and said solution is aseptically added to said vial.

20. The method of claim 17 wherein said compound is a solid, water-soluble salt of bis-1,2-(dimethylphosphino)ethane or *o*-phenylenebis(dimethylarsine), the halide is bromide or chloride, and the 99mTc-containing solution comprises a physiological saline solution of 99mTc-pertechnetate.

21. The method of claim 17 wherein said solution is substantially free of organic solvents.

22. The method of claim 17 wherein said solution further comprises ethanol or propylene glycol.

23. The method of claim 17 wherein said heating step is carried out at a temperature of at least 80°C.

24. The method of claim 17 wherein said heating step is carried out at a temperature greater than 100°C.

25. The method of claim 17 wherein the pH is maintained in the range of from 1.0 to 3.0 during the admixing and heating steps.

26. The method of claim 25 further comprising the step of adjusting the pH into the range of 4.0 to 9.0 before injecting said complex into a mammal, and scanning the mammal using radioscintigraphy imaging apparatus.

27. A pharmaceutical formulation comprising a compound of formula A or B as defined in claim 1, pharmaceutically acceptable salts of the compound in association with a pharmaceutically carrier.

28. A compound of formula A or B and pharmaceutically acceptable salts thereof for use in nuclear medicine.

29. A compound of formula A or B and pharmaceutically acceptable salts thereof for use in visualizing the heart or the hepatobiliary system of a mammal by radioscintigraphy.

30. The cationic complex formed by the method of claim 17 for use in visualizing the heart or hapatobiliary system of a mammal by radioscintigraphy.

## Patentansprüche

1. Normalerweise feste, hydrophile Verbindung, die in der Lage ist, mit Tc-99m unter Bindung eines kationischen Komplexes eine Bildung einzugehen, die aus den folgenden Formeln ausgewählt ist

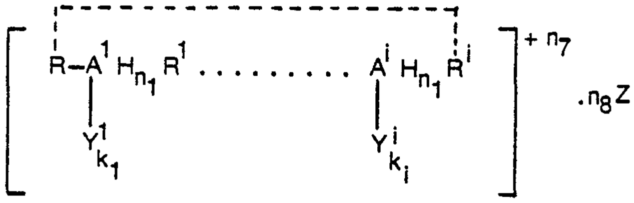

(A)

und

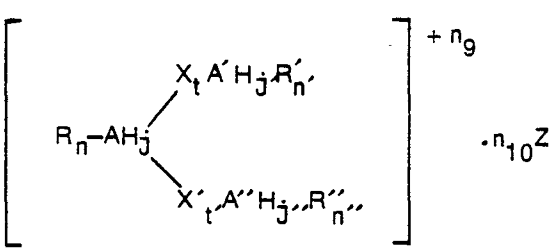

(B)

worin:

R, R', R'', $R^1$, $R^2$, $R^3$, $R^4$, $R^5$ und $R^6$ jeweils ausgewählt sind aus Wasserstoff, einer Alkylgruppe mit 1—6 Kohlenstoffatomen, Aryl, Alkylaryl, Arylalkyl, Monocycloalkyl und Polycycloalkyl; und worin R und R' in der Formel (A) zur Bildung einer cyclischen Verbindung zusammen genommen werden können;

A, A', A'', $A^1$, $A^2$, $A^3$, $A^4$, $A^5$ und $A^6$ Donoratome bedeuten, wobei jedes eine freies Elektronenpaar zur Aufnahme eines Protons zur Bildung eines geladenen Liganden zur Verfügung hat, und jedes in der Lage ist, mit Tc-99m zur Bildung eines kationischen Komplexes zu komplexieren, und unabhängig voneinander aus P, As, N, O, S, Sb, Se und Te ausgewählt ist.

$Y^1$, $Y^2$, $Y^3$, $Y^4$, $Y^5$ und $Y^6$ jeweils unabhängig voneinander aus Wasserstoff oder Alkyl, Aryl, Alkylaryl, Arylalkyl, Monocycloalkyl und Polycycloalkyl ausgewählt sind;

X und X' Alkylgruppen mit 1—6 Kohlenstoffatomen bedeuten;

Z ein Anion ist;

i eine ganze Zahl von 1 bis 6 bedeutet;

j, j' und j'' unabhängig voneinander 0 oder 1 bedeuten;

$k_1$, $k_2$, $k_3$, $k_4$, $k_5$ und $k_6$ unabhängig voneinander 0 oder 1 bedeuten;

t und t' unabhängig voneinander 0 oder 1 bedeuten;

n, n' und n'' unabhängig voneinander die ganze Zahl 1 oder 2 bedeuten;

$n_1$, $n_2$, $n_3$, $n_4$, $n_5$ und $n_6$ unabhängig voneinander 0 oder 1 bedeuten;

$n_7$ und $n_8$ jeweils eine ganze Zahl von 1 bis 6 bedeuten;

$n_9$ und $n_{10}$ jeweils eine ganze Zahl von 1 bis 3 bedeuten.

2. Verbindung nach Anspruch 1, worin Z von Schwefelsäure, Phosphorsäure, Perchlorsäure, Salpetersäure oder Borsäure stammt.

3. Verbindung nach Anspruch 1 mit der Formel:

$$R(AHjR'n')_n^{+n} \,.\, n''Z$$

worin R Ethyl oder Phenyl, A Phosphor oder Arsen, R' eine Alkylgruppe mit 1 bis 6 Kohlenstoffatomen, Z ein Anion, j 0 oder 1, n, n' und n'' 1 oder 2 bedeuten.

4. Verbindung nach Anspruch 3, worin Z Bisulfat, Biphosphat oder Tetrafluoroborat bedeutet.

5. Verbindung nach Anspruch 1 mit der Formel:

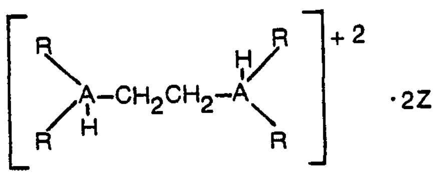

worin

Z ein Anion; A P oder As; und jeder Rest R unabhängig voneinander H, eine Alkylgruppe mit 1 bis 6 Kohlenstoffatomen oder Phenyl bedeutet.

6. Verbindung nach Anspruch 5, worin Z Bisulfat, Biphosphat, Nitrat, Tetrafluoroborat oder Perchlorat bedeutet.

7. Verbindung nach Anspruch 5, worin A Phosphor bedeutet.

8. Verbindung nach Anspruch 5, worin A Phosphor und R Methyl oder Ethyl bedeutet.

9. Verbindung nach Anspruch 5 mit der Formel:

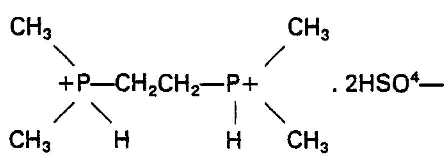

10. Verbindung nach Anspruch 1 mit der Formel:

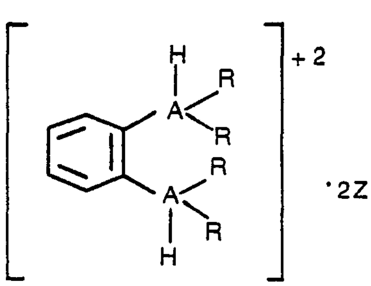

worin A P oder As und jeder Rest R unabhängig voneinander H, oder eine Alkylgruppe mit 1 bis 6 Kohlenstoffatomen und Z ein Anion bedeutet.

11. Verbindung nach Anspruch 10, worin Z Bisulfat, Biphosphat, Nitrat, Tetrafluoroborat oder Perchlorat bedeutet.

12. Verbindung nach Anspruch 10, worin A Phosphor bedeutet.

13. Verbindung nach Anspruch 10, worin A Phosphor und R Methyl oder Ethyl bedeutet.

14. Verbindung nach Anspruch 10 mit der Formel:

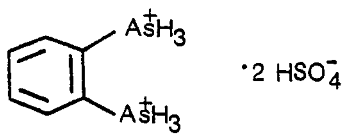

15. Reagentiensatz zur Herstellung einer radiopharmazeutischen Zubereitung, enthaltend ein hermetisch verschlossenes, sterilisiertes Fläschchen, das eine Verbindung nach einem der Ansprüche 1 bis 14 enthält.

16. Reagentiensatz nach Anspruch 15, worin das Fläschchen weiterhin ein Halogenidion oder ein Thiocyanato-, Cyano-, Cyanato-, Selenocyanato-, Fulminato-Ion und die Isoformen dieser Ionen; ein Azidoion oder ein Hydroxidion enthält.

17. Verfahren zur Herstellung eines kationischen Komplexes, enthaltend Tc-99m für die radioscintigrafische Abbildung, wobei das Verfahren aus der Zumischung der Verbindung nach Anspruch 1 mit einer wäßringen Lösung, die Tc-99m enthält, in Gegenwart eines Halogenidions oder eines Thiocyanato-, Cyano-, Cyanato-, Selenocyanato-, Fulminato-Ion und den Isoformen dieser Ionen, oder eines Azido- oder Hydroxidions sowie Erhitzung der Mischung zur Bildung eines stabilen Komplexes, besteht.

18. Verfahren nach Anspruch 17, worin das Verfahren unter aseptischen Bedingungen in einem geschlossenen System durchgeführt wird.

19. Verfahren nach Anspruch 18, worin die Verbindung in einem sterilen, hermetisch verschlossenen Fläschchen enthalten ist und die Lösung aseptisch zu dem Fläschchen zugegeben wird.

20. Verfahren nach Anspruch 17, worin die Verbindung ein festes, wasserlösliches Salz von Bis-1,2-(dimethylphosphino)-ethan oder *o*-Phenylen-bis(dimethylarsin) ist, das Halogenid Bromid oder Chlorid ist, und die 99mTc-enthaltende Lösung eine physiologische Salzlösung von 99mTc-Pertechnetat enthält.

21. Verfahren nach Anspruch 17, worin die Lösung im wesentlichen von organischen Lösungsmitteln frei ist.

22. Verfahren nach Anspruch 17, worin die Lösung weiterhin Ethanol oder Propylenglykol enthält.

23. Verfahren nach Anspruch 17, worin der Erhitzungsschritt bei einer Temperatur von mindestens 80°C durchgeführt wird.

24. Verfahren nach Anspruch 17, worin der Erhitzungsschritt bei einer Temperatur von mehr als 100°C durchgeführt wird.

25. Verfahren nach Anspruch 17, worin der pH-Wert im Bereich von 1,0 bis 3,0 während der Zusammenmischungs- und Erhitzungsschritte gehalten wird.

26. Verfahren nach Anspruch 25, worin weiterhin der pH-Wert im Bereich von 4,0 bis 9,0 vor der Injektion des Komplexes in ein Säugetier eingestellt wird und das Säugetier unter Verwendung eines Radioscintigrafie-Abbildungsapparates untersucht wird.

27. Pharmazeutische Formulierung, enthaltend eine Verbindung der Formal A oder B, wie in Anspruch 1 definiert, ihre pharmazeutisch verträglichen Salze in Verbindung mit einem pharmazeutisch verträglichen Träger.

28. Verbindung der Formel A oder B und ihre pharmazeutisch verträglichen Salze zur Verwendung in der Nuklearmedizin.

29. Verbindung der Formel A oder B und ihre pharmazeutisch verträglichen Salze zur Verwendung in der Sichtbarmachung des Herzens oder des hepatobiliären Systems eines Säugetiers durch Radioscintigrafie.

30. Kationischer Komplex, erzeugt durch das Verfahren nach Anspruch 17 zur Verwendung in der Visualisierung des Herzens oder des hepatobiliären Systems eines Säugetiers durch Radioscintigrafie.

**Revendications**

1. Un composé hydrophile, normalement solide, capable de se lier avec Tc-99m pour former un complexe cationique et ayant une formule choisie parmi:

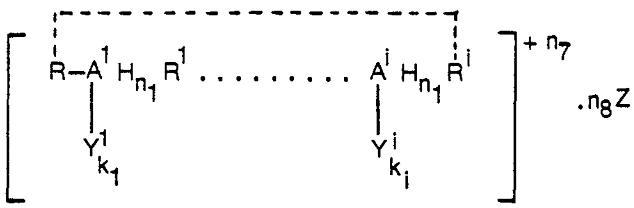

(A)

et

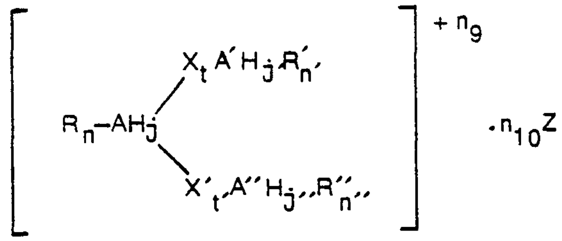

(B)

où:

R, R′, R′′, $R^1$, $R^2$, $R^3$, $R^4$, $R^5$ et $R^6$ sont choisis chacun parmi l'hydrogène et les groupes alcoyle ayant 1—6 atomes de carbone, aryle, alcoylaryle, arylalcoyle, monocycloalcoyle et polycycloalcoyle; et R plus $R^i$ dans la formule (A) peuvent être pris ensemble pour former un composé cyclique;

A, A′, A′′, $A^1$, $A^2$, $A^3$, $A^4$, $A^5$ et $A^6$ sont des atomes donneurs, ayant chacun une paire d'électrons libres, disponible pour accepter un proton afin de donner un ligand chargé et ayant la capacité de se complexer avec Tc-99m pour former un complexe cationique, et sont choisi indépendamment parmi P, As, N, O, S, Sb, Se et Te;

$Y^1$, $Y^2$, $Y^3$, $Y^4$, $Y^5$ et $Y^6$ sont choisi chacun indépendamment parmi l'hydrogène ou les groupes alcoyle, aryle, alcoylaryle, arylalcoyle, monocycloalcoyle, et polycycloalcoyle;

X et X′ sont des groupes alcoyle ayant 1—6 atomes de carbone;

Z est un anion;

i est un nombre entier de 1 à 6;

j, j′ et j′′ sont chacun indépendamment 0 ou 1;

$k_1$, $k_2$, $k_3$, $k_4$, $k_5$ et $k_6$ sont chacun indépendamment 0 ou 1;

t et t′ sont indépendamment 0 ou 1;

n, n′ et n′′ sont chacun indépendamment le nombre entier 1 ou 2;

$n_1$, $n_2$, $n_3$, $n_4$, $n_5$ et $n_6$ sont indépendamment 0 ou 1;

$n_7$ et $n_8$ sont chacun un nombre entier de 1 à 6;

$n_9$ et $n_{10}$ sont chacun un nombre entier de 1 à 3.

2. Le composé de la revendication 1, dans lequel Z est dérivé d'acide sulfurique, d'acide phosphorique, d'acide perchlorique, d'acide nitrique ou d'acide borique.

3. Le composé de la revendication 1 ayant la formule

$$R(AHjR'n')_n^{+n} \,.\, n''Z$$

où R est un groupe éthyle ou phényle, A est du phosphore ou de l'arsenic, R′ est un groupe alcoyle ayant de 1 à 6 atomes de carbone, Z est un anion, j est 0 ou 1; n, n′ et n′′ sont 1 ou 2.

4. Le composé de la revendication 3 dans lequel Z est un anion bisulfate, biphosphate ou tétrafluoroborate.

5. Le composé de la revendication 1 ayant la formule:

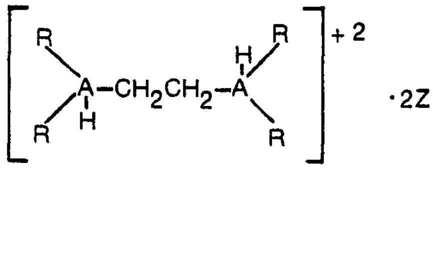

où

Z est un anion;

A est P ou As; et

chaque R indépendamment est H, un groupe alcoyle ayant de 1 à 6 atomes de carbone ou phényle.

6. Le composé de la revendication 5 dans lequel Z est un anion bisulfate, biphosphate, nitrate, tétrafluoroborate ou perchlorate.

7. Le composé de la revendication 6 dans lequel A est du phosphore.

8. Le composé de la revendication 5 dans lequel A est du phosphore et R est un groupe méthyle ou éthyle.

9. Le composé de la revendication 5 ayant la formule

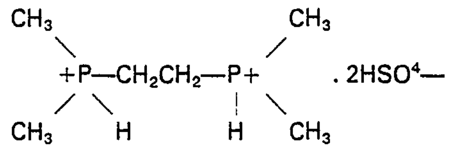

10. Le composé de la revendication 1 ayant la formule

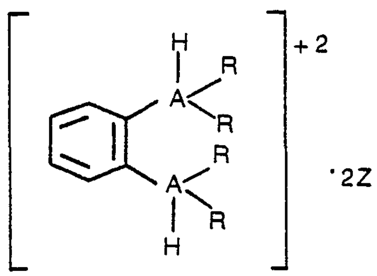

où A est P ou As; et chaque R est indèpendamment H ou un groupe alcoyle ayant de 1 à 6 atomes de carbone et Z est un anion.

11. Le composé de la revendication 10 dans lequel Z est un anion bisulfate, biphosphate, nitrate, tétrafluoroborate ou perchlorate.

12. Le composé de la revendication 10 dans lequel A est du phosphore.

13. Le composé de la revendication 10 dans lequel A est du phosphore et R est un groupe méthyle ou éthyle.

14. Le composé de la revendication 10 ayant la formule

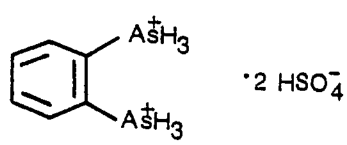

15. Un nécessaire pour préparer une préparation radiopharmaceutique comprenant un flacon fermé, stérilisé, contenant un composé selon l'une quelconque des revendications précédentes.

16. Le nécessaire de la revendication 15 dans lequel le flacon contient aussi un ion halogénure ou un ion thiocyanato, cyano, cyanato, sélénocyanato, fulminato et les formes iso de ces ions, un ion azido ou un ion hydroxyde.

17. Un procédé de préparation d'un complexe cationique comprenant du Tc-99m pour formation d'images radioscintigraphiques, ce procédé comprenant le mélange du composé de la revendication 1 avec une solution aqueuse contenant du Tc-99m en présence d'un ion halogénure ou d'un ion thiocyanato, cyano, cyanato, sélénocyanato, fulminato et la forme iso de ces ions, ou un ion azido ou hydroxyde, et le chauffage du mélange pour former un complexe stable.

18. Le procédé de la revendication 17 mis en oeuvre dans des conditions aseptiques et dans un système fermé.

19. Le procédé de la revendication dans lequel ledit composé est contenu dans un flacon stérile, scellé, et ladite solution est ajoutée aseptiquement dans ce flacon.

20. Le procédé de la revendication 17 dans lequel ledit composé est un sel solide, soluble dans l'eau de bis-1,2-(diméthylphosphino)éthane ou d'*o*-phénylènebis(dimèthylarsine), l'halogènure est du bromure ou du chlorure, et la solution contenant du 99mTc comprend une solution saline physiologique de 99mTc-pertechnétate.

21. Le procédé de la revendication 17 dans lequel ladite solution est sensiblement exempte de solvants organiques.

22. Le procédé de la revendication 17 dans lequel ladite solution comprend en outre de l'éthanol ou du propylène-glycol.

23. Le procédé de la revendication 17 dans lequel l'étape de chauffage est conduite à une température d'au moins 80°C.

24. Le procédé de la revendication 17 dans lequel l'étape de chauffage est conduite à une température supérieure à 100°C.

25. Le procédé de la revendication 17 dans lequel le pH est maintenu entre 0,1 et 3,0 durant les étapes de mélange et de chauffage.

26. Le procédé de la revendication 25 comprenant en outre l'étape consistant à régler le pH dans l'intervalle de 4,0 à 9,0 avant injection du complexe dans un mammifère, et l'examen du mammifère en utilisant un appareil de formation d'images par radioscintigraphie.

27. Une composition pharmaceutique comprenant un composé de formule A ou B tel que défini dans la revendication 1, des sels pharmaceutiquement acceptables du composé en association avec un véhicule pharmaceutique.

28. Un composé de formule A ou B et ses sels pharmaceutiquement acceptables pour utilisation en médicine nucléaire.

29. Un composé de formule A ou B et ses sels pharmaceutiquement acceptables pour utilisation dans la visualisation du coeur ou du système hépatobiliaire d'un mammifère par radioscintigraphie.

30. Le complexe cationique formé par le procédé de la revendication 17 pour utilisation dans la visualisation du coeur ou du système hépatobiliaire d'un mammifère par radioscintigraphie.